# EUROPEAN PATENT APPLICATION

(11) **EP 1 825 840 A1**
(43) Date of publication of application: **29.08.2007**
(21) Application number: 07003440.0
(22) Date of filing: 19.02.2007
(51) Int. Cl.: A61F 11/00, A61M 5/28, A61M 39/04

(54) **Minute quantity medicine administration set**

(30) Priority: 24.02.2006 JP 2006048556
(71) Applicant: NIPRO CORPORATION, Kita-ku, Osaka-shi, Osaka-fu, 531-8510 (JP)
(72) Inventor: Murakami, Mitsuo, Osaka-shi Osaka-fu, 531-8510 (JP); Namba, Ryohei, Osaka-shi Osaka-fu, 531-8510 (JP)
(74) Representative: Banzer, Hans-Jörg

(57) **Abstract**

A minute quantity medicine administration set including an injection needle (1) having a cannula (11) and a needle hub (12) ; a minute quantity administration device (2) equipped with an injection needle connector (21) having a connection part (211) for connecting with the needle hub (12), a tube member (22) connected to the injection needle connector (21), and a piercing needle connector (23) having a tube member connection part (231) for connecting with the tube member (22) at its front end and having a hollow piercing needle connection part (232) for connecting with a piercing needle (31) of a transfer needle (3) at its base end, the transfer needle (3) in which the hollow piercing needle (31) and a syringe connection part (32) are provided respectively on opposite sides of a disc-like hub (33) on the same axis, a cylindrical vial mounting part (34) extending concentrically with the piercing needle (31) is provided at an outer edge of the disk-like hub (33) beyond a front end of the piercing needle (31), the piercing needle (31) communicating with the syringe connection part (32); and a syringe (4) equipped with a connection part (41) for connecting to the transfer needle (3), a barrel (42) connected to the transfer needle connection part (41), and a plunger (43) inserted into the barrel (42).

## Description

### BACKGROUND OF THE INVENTION

### 1. Field of the Invention

The present invention relates to a minute quantity medicine administration set capable of precisely and safely administering a medicine, such as an organism tissue adhesive, or bio-adhesive, in a minute quantity.

### 2. Prior Art

For many years, an eardrum hole closing technique or an eardrum formation technique has been performed on a chronic otitis media and an eardrum perforated by a lesion. Generally, in the eardrum formation technique, a graft material is inserted into a tympanum and is stuck fast to an inside of a perforated edge, and then, the graft material is bonded to the perforated edge of the eardrum by dropping a fibrin adhesive on it. As a minute quantity administration device for administering a bio-adhesive such as the fibrin adhesive a bio-adhesive administration device having a tube which is capable of being pinched or squeezed is provided between a syringe and a deformable nozzle and has parts for connecting respectively with the syringe and the nozzle at its ends has been proposed (JP-A-2001-17437).

However, in the bio-adhesive administration device having a tube, which is capable of being squeezed, interposed between a syringe and a deformable nozzle and has parts at its ends for connecting respectively with the syringe and the nozzle, because the operations (i) - (iv) mentioned below must be performed in order to administer the medicine, a needle that must be attached for transferring the medicine and, thereafter, removed is poor in operation efficiency.
(i) The needle for transferring the medicine from a vial is attached to the syringe.
(ii) The needle is pierced into a rubber plug of the vial, then the medicine is sucked from the vial into the syringe.
(iii) The needle for transferring is removed.
(iv) A tube member is attached to the syringe.

Further, since a conventional injection needle is used as the needle for transferring the medicine, there is a fear that coring (separation of a rubber piece) occurs in a case of slantingly piercing the rubber plug of the vial by the needle.
Therefore, an object of the present invention is to provide a minute quantity medicine administration set avoiding the use of a needle for transferring the medicine, which is a conventional injection needle.

### SUMMARY OF THE INVENTION

According to the present invention, this object is achieved by a minute quantity medicine administration set as defined in claim 1. The dependent claims define preferred and advantageous embodiments of the invention.

As a result of being dedicated to various studies in order to solve the above problems, the present inventors found that bio-adhesive accommodated in a syringe can be transferred to a minute quantity administration device through a cap member and a transfer needle by providing a cap member capable of being pierced with a transfer needle mentioned below in a piercing needle connection part of a minute quantity administration device, and can be discharged from an injection needle connection part by squeezing a tube member of the minute quantity administration device with the fingers, and can be administered to a diseased part from the injection needle, thereby, the inventors attained the present invention.

That is, the present invention in a first aspect is a minute quantity medicine administration set comprising:
(A) an injection needle having a cannula and a needle hub,
(B) a minute quantity administration device equipped with an injection needle connector having a connection part for connecting with the needle hub, a tube member connected to the injection needle connector, and a piercing needle connector which has a tube member connection part at its front end connected to the tube member and has a piercing needle connection part at its base end for connecting with a hollow piercing needle of a transfer needle,
(C) the transfer needle in which the hollow piercing needle and a syringe connection part are provided respectively on opposite sides of a disc-like hub on the same axis, a cylindrical vial mounting part extending concentrically with the piercing needle is provided at an outer edge or periphery of the hub beyond a front end of the piercing needle, and the piercing needle communicates with the syringe connection part, and
(D) a syringe provided with a transfer needle connection part for connecting with the transfer needle, a barrel connected to the transfer needle connection part, and a plunger inserted in the barrel.

A second aspect of the present invention is the minute quantity medicine administration set of the first aspect, wherein the piercing needle connection part has additionally a cap member covering the piercing needle connection part.

A third aspect of the present invention is the minute quantity medicine administration set of the first aspect, wherein the piercing needle connection part has a female thread on the outer periphery of its base end and additionally is provided with a hollow member having a male thread meshing with the female thread provided in the inner periphery of a cylindrical member and, a rubber member which can be pierced with the piercing needle is included in a bottom part of the hollow member.

A fourth aspect of the present invention is the minute quantity medicine administration set of the first aspect, wherein the piercing needle connection part has a check valve in its base end into which the piercing needle can be inserted and additionally a connector tube adapted to cover both the piercing needle connection part and the piercing needle is provided for connecting the piercing needle connection part and the piercing needle.

In the present invention, there is obtained the minute quantity medicine administration set capable of precisely and safely administering a medicine, such as a bio-adhesive, in the minute quantities without using a needle for transferring the medicine, which is a conventional injection needle.
The invention will now be described in more detail with reference to the accompanying drawings, in which:
Fig. 1 is a longitudinal sectional view showing each component constituting the minute quantity medicine administration set of the present invention.
Fig. 2 is a sectional view showing another embodiment of a minute quantity administration device of the present invention.
Fig. 3 is a sectional view showing another embodiment of the minute quantity administration device of the present invention.
Fig. 4 is a sectional view showing a syringe and a transfer needle to which the minute quantity administration device shown in Fig. 2 is connected.
Fig. 5 is a sectional view showing a syringe and a transfer needle to which the minute quantity administration device shown in Fig. 3 is connected.
Fig. 6 is a longitudinal sectional view showing procedures for using the minute quantity medicine administration set of the present invention.
Fig. 7 is a view illustrating a method of using the minute quantity administration device connected to an injection needle.

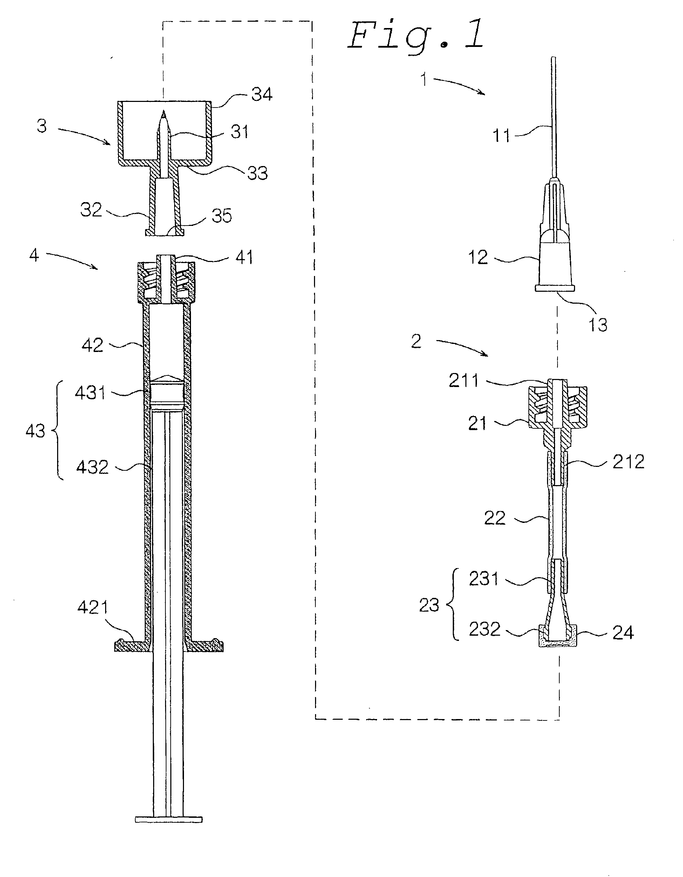

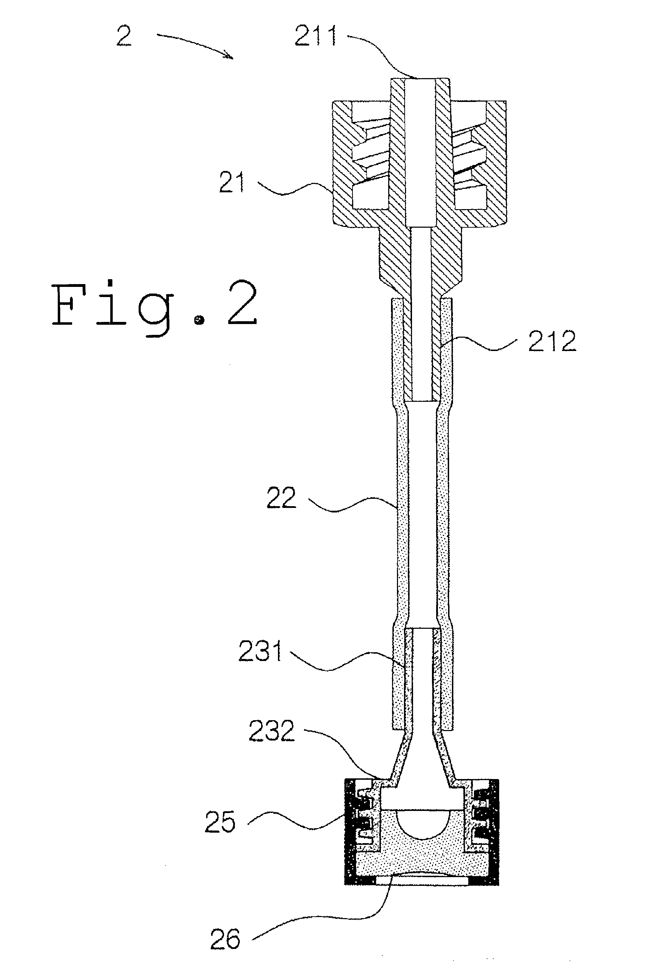

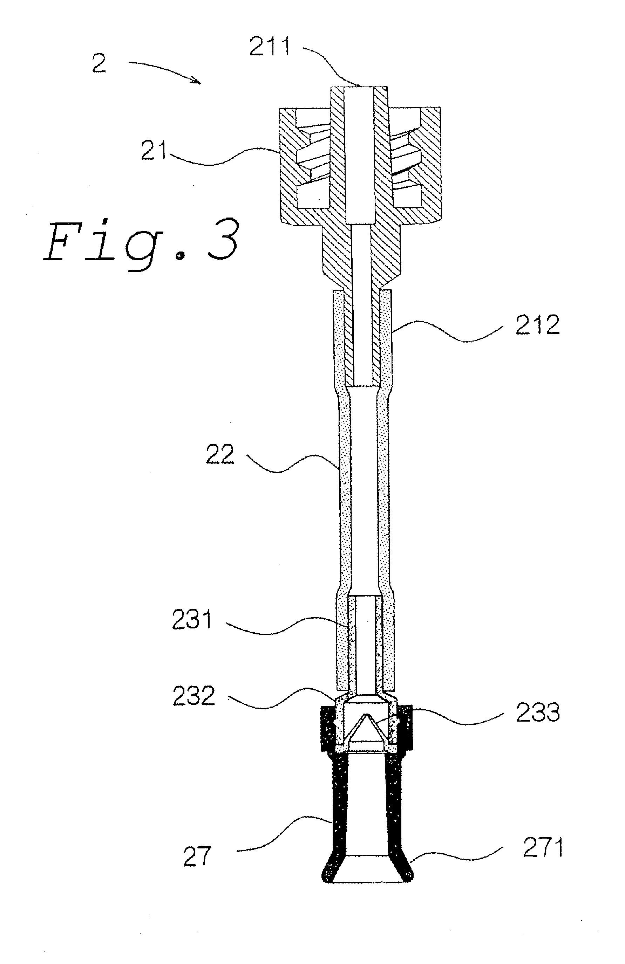

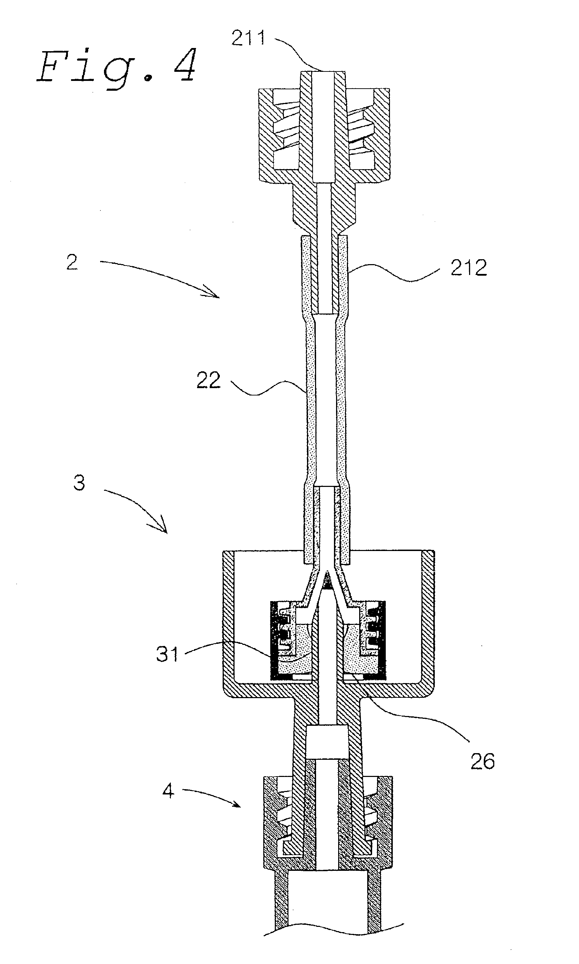

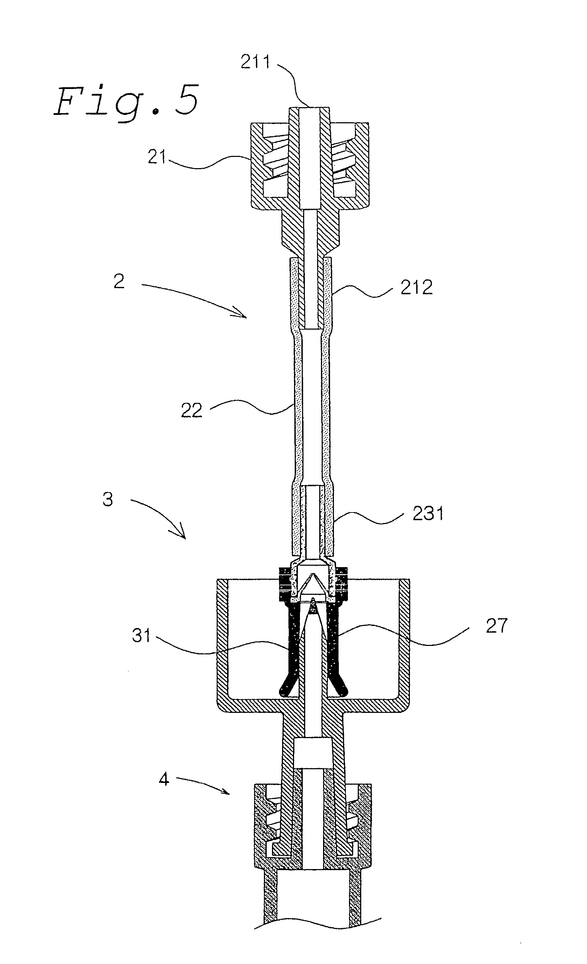

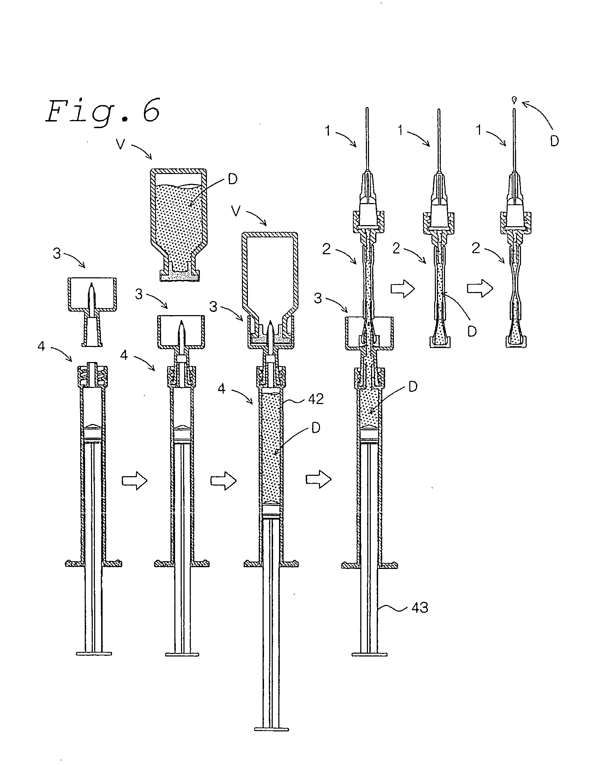

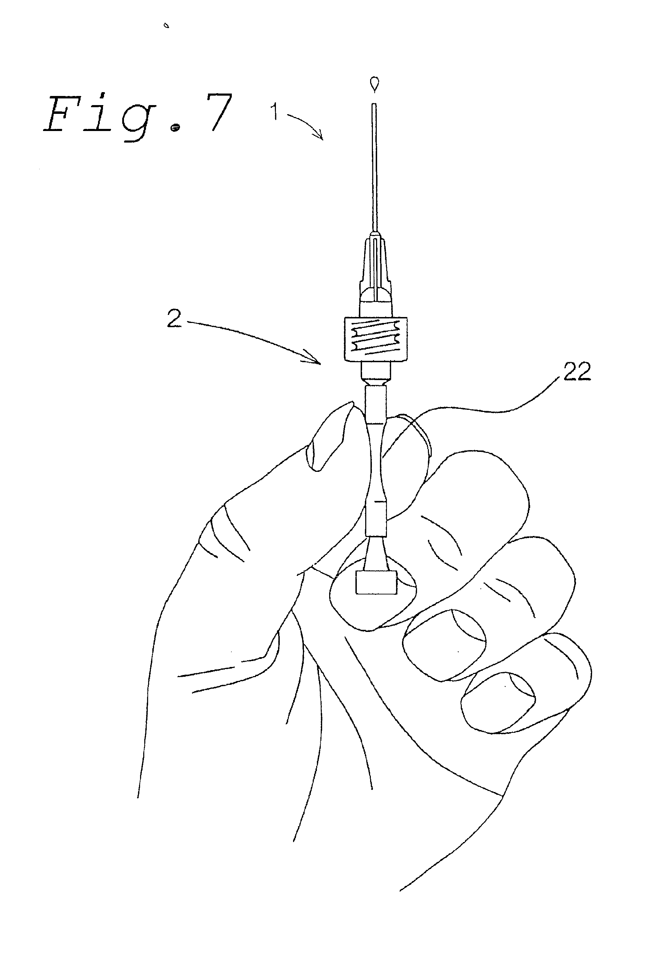

Next, the minute quantity medicine administration set of the present invention is explained on the basis of the drawings. Fig. 1 is a longitudinal sectional view showing each component comprising the minute quantity medicine administration set of the present invention, and Fig. 2 and Fig. 3 are sectional views showing other embodiments of the minute quantity administration device of the invention. Fig. 4 and Fig. 5 are sectional views showing a syringe and transfer needle to which the medicine minute quantity administration devices shown in Fig. 2 and Fig. 3 are connected. Fig. 6 is a longitudinal sectional view showing procedures for using the minute quantity medicine administration set of the present invention, and Fig. 7 is a view illustrating a method of using the medicine minute quantity administration device to which an injection needle has been connected.

The minute quantity medicine administration set of the present invention has an injection needle (1), a minute quantity administration device (2), a transfer needle (3), and a syringe (4).

In order to use the minute quantity medicine administration set, the transfer needle (3) and the syringe (4) are connected first, and the injection needle (1) and the minute quantity administration device (2) are connected. Subsequently, a vial in which the medicine to be administered is accommodated is connected to the transfer needle (3), and the medicine is infused into a barrel of the syringe (4) from the vial via the transfer needle (3). After the vial is removed from the transfer needle, the medicine is delivered to the minute quantity administration device (2) to which the injection needle (1) has been connected, the minute quantity administration device (2) is detached from the transfer needle (3), and the medicine having been delivered to the minute quantity administration device (2) is pushed out by pinching or squeezing the tube member 22, thereby administering the medicine to an object from the injection needle (1) (refer to Fig. 6).

An injection needle 1 of the minute quantity administration device in the present invention has a cannula 11 and a needle base 12. It is desirable that the cannula 11 is formed with no cutting edge, can be freely and precisely bent to a predetermined shape, and moreover has a characteristic of keeping intact a bent shape. As a material for forming the cannula 11, an annealed stainless steel (SUS 304 and the like) is desirable. The needle hub 12 is similar to that of a general injection needle, which is formed generally by a metal or a thermoplastic resin, e.g., polypropylene, polyethylene, methylpentene polymer, polycarbonate and the like, and is equipped with a fit part 13 for connecting to a minute quantity administration device 2. The fitting part 13 cooperates with a connection part 211 of an injection needle connection tool 21. The cannula 11 is insertion-fixed to a side opposite to the fitting part 13 of the needle hub 12.

The minute quantity administration device 2 in the invention has an injection needle connector 21 having a connection part 211 for connecting with the needle hub 12, a tube member 22 for communicating with the injection needle connector 21 in its front end, and comprises a piercing needle connector 23 having a tube member connection part 231 for connecting with the tube member 22, and a piercing needle connection part 232 for connecting with a piercing needle 31 of a transfer needle 3 in its base end.

The injection needle connector 21 has the connection part 211 for connecting with the needle hub 12 on its front end. A shape of the connection part 211 is suitably selected according to the type of needle hub 12 of the injection needle 1, e.g., a luer tip and a cylindrical luer lock part surrounding the luer tip, and it can be meshed with the needle hub 12. In a base end of the injection needle connector 21, a thin tubular part 212 is provided so as to be capable of communicating with one end of the tube member 22.
The tube member 22 has respective connection parts at its end for connecting with the injection needle connector 21 and the piercing needle connector 23. The tube member is a hollow tube for forcing liquid out when it is pinched, and which is formed of a soft vinyl chloride resin, a rubber elastic body, e.g., natural rubber, butyl rubber, halogenated butyl rubber, isoprene rubber, silicone rubber, and the like. Inner and outer diameters and a length of the tube member are not limited especially if it can dispense a minute quantity of liquid.

The piercing needle connector 23 in Fig. 1 has the connection part 231 for connecting with the tube member 22 in its front end, and the piercing needle connection part 232 for connecting with the transfer needle 3 in its base end. The piercing needle connection part 232 has, as an example, a cap member 24 covering the piercing needle connection part 232 (Fig. 1, Fig. 6).

The cap member 24 has such a function that, after the liquid has been delivered to the tube member 22 by piercing with the piercing needle 31 of the transfer needle 3, even when the piercing needle 31 is pulled out from the cap member, the liquid will be held inside the tube member 22 and, even when the tube member 22 is pinched, the liquid will not leak. Although there can be enumerated, as the material of the cap member, an elastic material such as, e.g., natural rubber, butyl rubber, halogenated butyl rubber, isoprene rubber and silicone rubber, the material is not especially limited.

The injection needle connector 21 and the piercing needle connector 23 are formed of a usually used thermoplastic resin, e.g., polypropylene, polyethylene, methylpentene polymer, polycarbonate, and the like.

As another embodiment of the minute quantity administration device 2 of the present invention, as shown in Fig. 2, the piercing needle connector has the connection part 231 for connecting with the tube member 22 at its front end, and has a female thread on the outer periphery of its base end. The female thread meshes with a hollow member 25 which has a male thread which cooperates with the female thread in an inner periphery of a cylindrical member at the base end. In the bottom part of the cylindrical member, there is included a rubber member 26 which can be pierced by the piercing needle 31. In order to be pierced in the center of the rubber member by the piercing needle 31, it is desirable that the thickness of the center of the rubber member 26 is thinner than that of the periphery of the rubber member. In this case, the above-mentioned cap member 24 covering the piercing needle connection tool 23 is unnecessary.

Fig. 4 is an illustration in which the minute quantity administration tool 2 shown in Fig. 2, the transfer needle 3 and the syringe 4 are connected, and the rubber member 26 and the piercing needle 31 are fluid-tightly connected.

As another embodiment of the minute quantity administration device 2 of the present invention, as shown in Fig. 3, there is one in which the piercing needle connector has the connection part 231 for connecting with the tube member 22 at its front end, and has a check valve 233 into which the piercing needle 31 can be inserted in its base end. A connector tube 27 for covering the piercing needle connection part 232 and the piercing needle 31 are provided, and thereby both can be connected. The check valve 233 opens when the liquid is infused into the minute quantity administration tool 2 from the syringe 4, but does not open when the tube member 22 is pinched, and thus has a function for preventing the liquid from leaking from the piercing needle connection part 232 side. Although, as a material for forming the check valve 233, an elastic material is enumerated, e.g., natural rubber, butyl rubber, halogenated butyl rubber, isoprene rubber and silicone rubber, the material is not especially limited. Further, the connector tube 27 is adapted to be fluid-tightly connected with the piercing needle connection part 232 through the check valve 233. Further, the base end of the connector tube 27 may have a flared end 271, which functions as a guide for connecting the piercing needle connection part 232 and the piercing needle 31. Although a material for forming the connector tube 27 can be a plastic resin, e.g., polypropylene, polyethylene, methylpentene polymer and polycarbonate, the material is not especially limited.

Fig. 5 is an illustration in which the minute quantity administration device 2 shown in Fig. 3 and the transfer needle 3 are connected, and the piercing needle 31 is fluid-tightly connected to the connector tube 27.

In the transfer needle 3 in the present invention, the hollow piercing needle 31 and a syringe connection part 32 are provided respectively in opposite sides on the same axis of a disc-like hub 33 and a cylindrical vial mounting part 34 extending concentrically with the piercing needle 31 beyond a front end of the piercing needle 31 is provided at an outer edge or periphery of the hub 33. A syringe mounting part 35 is provided in the syringe connection part 32, and can be joined with a transfer needle connection part 41 of the syringe 4.
A material for forming the transfer needle 3 is a usually used thermoplastic resin, e.g., polypropylene, polyethylene, methylpentene polymer, polycarbonate, and the like. The piercing needle 31 is adapted so as to be capable of piercing a rubber-made cap of the vial or the like, and it may be formed monolithically with the transfer needle 3, or it may be adapted to be attached to the transfer needle 3 consisting of a metal material.

A syringe 4 in the present invention has the transfer needle connection part 41 which is capable of being joined to the syringe mounting part 35 of the transfer needle 3, a barrel 42 connected to the transfer needle connection part 41, and a plunger 43 inserted into the barrel 42.
The shape of the transfer needle connection part 41 is suitably selected according to the type of the syringe mounting part 35 of the transfer needle 3, e.g., a luer tip and a cylindrical luer lock part surrounding the luer tip, and can mesh with the syringe mounting part 35.
The barrel 42 is a cylindrical member having a space for accommodating a medicine. A finger grip 421 which facilitates an operation pushing of the plunger 43 is provided on the base end side of the barrel.
The plunger 43 has a gasket 431 at its front end and a plunger rod 432 in its intermediate portion and, thereby medicine can be infused and delivered by a manual operation.
A material for forming the syringe 4 is a usually used thermoplastic resin, e.g., polypropylene, polyethylene, methylpentene polymer, polycarbonate, and the like.

In order to use the minute quantity medicine administration set of the present invention, as shown in Fig. 6 for instance, first, the transfer needle 3 and the syringe 4 are connected. Next, a vial V containing a medicine is connected to the transfer needle 3, and the medicine D is infused into the barrel 42 while passing through the transfer needle 3 from the vial V. After the vial V is removed from the transfer needle 3, the minute quantity administration device 2 connected to the injection needle 1 is connected to the transfer needle 3. The medicine D is delivered into the tube member 22 of the minute quantity administration device 2 by pushing the plunger 43 of the syringe 4. Subsequently, the minute quantity administration device 2 is removed from the transfer needle 3, and the tube member 22 is pinched by fingers, to thereby administer the medicine D from the injection needle 1 to the object portion from the injection needle 1.
A mode of pinching the tubular member 22 by the fingers is shown in Fig. 7.

In the present invention the medicine is not limited to a bio-adhesive such as fibrin adhesive, and various medicines which are to be administered to a localized area in a minute quantity can be utilized.

## Claims

1. A minute quantity medicine administration set comprising:
an injection needle (1) having a cannula (11) and a needle hub (12),
a minute quantity administration device (2) comprising an injection needle connector connection part (211) for connecting with the needle hub (12), a tube member (22) connected to the injection needle connector (21), and a piercing needle connector (23) which has at its front end a tube member connection part (231) connected to the tube member (22) and has at its base end a piercing needle connection part (232) for connecting with a piercing needle (31) of a transfer needle (3),
the transfer needle (3) in which the piercing needle (31) and a syringe connection part (32) are provided respectively on the same axis on opposite sides of a disc-like hub (33), and a cylindrical vial mounting part (34) extending concentrically with the piercing needle (31) is provided at an outer periphery of the disk-like hub (33) and extends beyond a front end of the piercing needle (31), and the piercing needle (31) communicates with the syringe connection part (32), and
a syringe (4) comprising a connection part (41) for connecting with the transfer needle (3), a barrel (42) connected at a the transfer needle connection part (41), and a plunger (43) inserted into the barrel (42).

2. A minute quantity medicine administration set according to claim 1, wherein the piercing needle connection part (232) includes a cap member (24) covering the piercing needle connection part (232).

3. A minute quantity medicine administration set according to claim 1 or claim 2, wherein the piercing needle connection part (232) has a female thread on the outer periphery of its base end, and additionally comprises a hollow cylindrical member (25) having a male thread meshing with the female thread in an inner periphery of said cylindrical member (25), and a rubber member (26) which can be pierced by the piercing needle (31) positioned in a bottom part of the hollow member (25).

4. A minute quantity medicine administration set according to any one of claims 1-3, wherein the piercing needle connection part (232) has a check valve (233) in its base end into which the piercing needle (31) can be inserted, and additionally comprises a connector tube (27) for covering both the piercing needle connection part (232) and the piercing needle (31) to thereby connect the piercing needle connection part (232) and the piercing needle (31).
